Europäisches Patentamt

European Patent Office

Office européen des brevets

(19)

(11) Veröffentlichungsnummer: **0 129 919**
**A1**

# (12) EUROPÄISCHE PATENTANMELDUNG

(21) Anmeldenummer: 84200556.3

(22) Anmeldetag: 18.04.84

(51) Int. Cl.⁴: **C 07 C 1/24**
**C 07 C 11/02, C 10 G 3/00**

(30) Priorität: 28.05.83 DE 3319456

(43) Veröffentlichungstag der Anmeldung:
02.01.85 Patentblatt 85/1

(84) Benannte Vertragsstaaten:
DE FR GB NL

(71) Anmelder: METALLGESELLSCHAFT AG
Reuterweg 14 Postfach 3724
D-6000 Frankfurt/M.1(DE)

(72) Erfinder: Buchold, Henning, Dr.
Erzbergerstrasse 3
D-6450 Hanau am Main(DE)

(72) Erfinder: Cornelius, Gerhard
Dornbachweg 2
D-6367 Karben 4(DE)

(72) Erfinder: Möller, Friedrich, Dr.
Breslauer Ring 15
D-6382 Friedrichsdorf(DE)

(72) Erfinder: Supp, Emil
Marburger strasse 7
D-6057 Dietzenbach(DE)

(74) Vertreter: Fischer, Ernst, Dr.
Reuterweg 14
D-6000 Frankfurt am Main 1(DE)

(54) Verfahren zur Herstellung von Olefinen mit 2 bis 5 C-Atomen aus Methanol und/oder Dimthylether.

(57) Das Einsatzmaterial, das üblicherweise Wasser enthält, wird bei Temperaturen von 200 bis 600°C und einem Druck von 1 bis 35 bar an einem Silicalite-Katalysator umgesetzt. Silicalite ist kristallines $SiO_2$ ohne Ionenaustauschfähigkeit.

EP 0 129 919 A1

Croydon Printing Company Ltd

METALLGESELLSCHAFT AG

Nr. 8980 LÖ

Frankfurt, 27.05.1983
-WGN/HSZ-

**0129919**

## Verfahren zur Herstellung von Olefinen mit 2 bis 5 C-Atomen aus Methanol und/oder Dimethylether

Die Erfindung betrifft ein Verfahren zur Herstellung von Olefinen mit 2 bis 5 C-Atomen aus Methanol, das man bei Temperaturen von 200 bis 600°C und einem Druck von 1 bis 35 bar an einem Silicium enthaltenden Katalysator umsetzt und ein olefinhaltiges Produkt abzieht.

Verfahren dieser Art sind aus den deutschen Offenlegungsschriften 27 55 229, 28 27 385 und 31 18 954 bekannt. Auch in den US-Patenten 4 052 479 und 4 062 905 ist die Herstellung kurzkettiger Olefine auf dem eingangs genannten Weg erläutert.

Den bekannten Verfahren ist gemeinsam, daß es sich bei den Katalysatoren um zeolithische Aluminiumsilikate handelt, die Ionenaustauscheigenschaften haben und durch ein definiertes, wenn auch variierbares $SiO_2/Al_2O_3$-Verhältnis gekennzeichnet sind. Bei den Umsetzungen an diesen Katalysatoren entsteht ein olefinhaltiges Produkt, dessen Hauptkomponente Äthylen ($C_2H_4$) ist.

Der Erfindung liegt die Aufgabe zugrunde, einen hoch wirksamen Katalysator für das eingangs genannte Verfahren bereitzustellen, der in der Lage ist, ein Produkt zu erzeugen, das mehr Propylen ($C_3H_6$) als Äthylen enthält. Erfindungsgemäß wird dies dadurch erreicht, daß als Katalysator kristallines Siliciumdioxid ohne Ionenaustauschfähigkeit (Silicalite) verwendet wird. Silicalite ist eine an sich bekannte Substanz, deren Herstellung im US-Patent 4 061 724 beschrieben ist.

2

Aus "Hydrocarbon Processing", November 1980, Seiten 139 bis 142, ist es bekannt, $C_2$- bis $C_4$-Olefine aus Synthesegas, einem Gasgemisch aus Kohlenmonoxid und Wasserstoff, mit eisendotiertem Silicalite als Katalysator zu erzeugen. Es war deshalb sehr überraschend zu finden, daß Silicalite Methanol und/oder Dimethylether mit hoher Selektivität zu Olefinen umsetzt und dies in der vorteilhaften Weise mit hohem Propylenanteil im Produkt. Ausgehend vom Stand der Technik wäre eher zu vermuten gewesen, daß Methanol in Gegenwart von Silicalite nur eine geringfügige Umwandlung in ein Kohlenwasserstoffgemisch mit 1 bis 10 C-Atomen erfahren würde, weil Silicalite im Gegensatz zu den Aluminiumsilikat-Zeolithen keine sauren Eigenschaften (H-Acidität) aufweist ("Hydrocarbon Processing" Nov. 1980, Seiten 139 bis 142). Silicalite ist reines $SiO_2$, das im Kristallgitter kein Aluminium enthält.

In "Hydrocarbon Processing", Nov. 1982, Seiten 117 bis 120, wird die Verwendung modifizierter Zeolithe für das eingangs genannte Verfahren beschrieben. Dabei beträgt die Betriebszeit zwischen zwei Regenerationen des Katalysators 11 Stunden. Die Regeneration wurde notwendig, da sich der Katalysator mit Ruß bedeckte und dadurch inaktiv wurde. Durch Regeneration wird der Kohlenstoff auf dem Katalysator in bekannter Weise oxidativ entfernt. Beim Verfahren der Erfindung mit dem Silicalite-Katalysator werden demgegenüber Betriebszeiten zwischen zwei Regenerationen von 1000 Stunden und mehr erreicht, ohne Einbußen an Umsatz und Selektivität. Diese langen Standzeiten sind für die Praxis und die Ökonomie des Verfahrens von größtem Vorteil.

Als Einsatzstoffe des Verfahrens können Methanol und, wie bei bekannten Verfahren, auch Dimethylether oder niedere Alkohole, z.B. Äthanol, verwendet werden.

Die Umsetzung von verdampftem Methanol oder von Dimethylether
am Silicalite-Katalysator erfolgt üblicherweise in Gegenwart
von Wasserdampf, wie dies auch für die Zeolith-Katalysatoren
bekannt ist. Nun haben aber Zeolith-Katalysatoren und auch
andere Ionenaustauscher u.a. den Nachteil, daß sich besonders
bei Temperaturen oberhalb 400°C in Gegenwart von Wasserdampf
nur kurze Betriebszeiten erreichen lassen. Auch in dieser
Hinsicht ist der Silicalite-Katalysator deutlich überlegen,
da er bei Temperaturen über 400°C und auch über 450°C und hohem
Wasserdampfgehalt im Reaktionsgemisch keine beschleunigte
Desaktivierung zeigt.

Man kann den Silicalite-Katalysator natürlich auch im Gemisch
mit anderen bekannten Katalysatoren, z.B. Zeolithen, verwenden.
Dadurch läßt sich das Produkt der Umsetzung modifizieren.
Besteht der Katalysator allein aus Silicalite, so enthält das
Produkt pro kg $C_2H_4$ mindestens 2,5 kg $C_3H_6$, was für die Praxis
sehr erwünscht ist. Hierbei ist vor allem an die Herstellung
von Dieselkraftstoff aus den Olefinen, bevorzugt $C_3H_6$ und $C_4H_8$,
zu denken. Diese Herstellung der Dieselkraftstoffe ist bereits
bekannt. Es wurde auch gefunden, daß die erzeugten Olefine
pro kg $C_2H_4$ mindestens 1 kg $C_4H_8$ enthalten. Demgegenüber
erzielt man mit einem zeolithischen Aluminiumsilikat-Katalysator ein Produkt, dessen Hauptkomponente Äthylen ist.

Die katalytische Wirksamkeit des Silicalite-Katalysators ist
hoch, so daß man die Umsetzung von Methanol im Katalysator-
Festbett durchführen und auf die Rückführung von Restgasen
und/oder Einsatzstoff verzichten kann. Dabei werden mindestens
80% des Methanols zu $C_2$- bis $C_5$-Olefinen umgesetzt. Vor allem
wenn man das Produkt weiterverarbeitet, stört es nicht, wenn
es noch etwas Methanol enthält.

Das erfindungsgemäße Verfahren wird bevorzugt in 3 Varianten
in die Praxis umgesetzt:

a) Man verwendet einen Röhrenreaktor, in dessen Röhren sich der Katalysator befindet, wobei die Reaktionswärme an ein flüssiges Kühlmittel, z.B. eine Salzschmelze, abgegeben wird (quasi-isotherme Fahrweise), oder

b) in adiabater Fahrweise, wobei die Reaktionswärme im Produktgas verbleibt und dessen Temperatur erhöht. Der auch bei hohen Temperaturen sehr stabile Silicalite-Katalysator ist hierfür gut geeignet.

c) Es ist auch ein zweistufiges Verfahren möglich, wobei man Methanoldampf in an sich bekannter Weise (US Patent 3 998 899) an einem Dehydratisierungskatalysator (z.B. $Al_2O_3$) bei Temperaturen von etwa 200 bis 400°C zu einem vor allem Dimethylether enthaltenden Zwischenprodukt umsetzt und dieses Zwischenprodukt dann über den Silicalite-Katalysator leitet.

## Beispiel 1

Im Labormaßstab werden stündlich 0,3 kg eines Gemisches aus Methanol und Wasser (Gewichtsverhältnis 1 : 3) bei einem Druck von 1 bar und bei einer Eintrittstemperatur von 480°C am Silicalite-Katalysator bei einmaligem Durchgang umgesetzt. 0,3 kg des Katalysators befinden sich in einem Rohr von 1,5 m Länge mit einem Innendurchmesser von 25 mm. Der Katalysator, S-115 von Union Carbide Corp., besteht aus zylindrischen Extrudaten einer ungefähren Dicke von 1,6 mm. Das den Katalysator enthaltende Rohr wird durch eine Salzschmelze indirekt gekühlt.

Das Reaktionsprodukt, das den Katalysator mit 480°C verläßt und praktisch frei von Methanol ist, wird in einem Wasserkühler indirekt auf etwa 25°C gekühlt; in einem Abscheider trennen sich die Gasphase und die Flüssigphase. Die Gasphase stellt das gewünschte Produkt dar, die Flüssigphase besteht aus Wasser und höhersiedenden Kohlenwasserstoffen ($C_5^+$). Das Gesamtprodukt enthält Kohlenwasserstoffe folgender Art:

| | |
|---|---|
| Äthylen | 11,0 Gew.% |
| Propylen | 50,2 " |
| Butylene | 20,6 " |
| zusammen | 81,8 Gew.% |

und dazu $C_1$- bis $C_4$-Paraffine  5,1 Gew.%
sowie $C_5^+$-Kohlenwasserstoffe  13,1 Gew.%.

Pro kg umgesetztem Methanol erhält man also 0,358 kg Äthylen, Propylen und Butylenen, das sind 81,8% der theoretisch möglichen Ausbeute.

Beispiel 2

In der Anordnung und Verfahrensführung des Beispiels 1 wird der Einfluß der Betriebsdauer auf die Olefinausbeute und den Methanolumsatz untersucht. Auch hier wird das Methanol-Wasser-Gemisch bei einmaligem Durchströmen des Reaktionsrohres und ohne Zwischenregenerieren des Katalysators umgesetzt.

| Betriebsdauer: | | 250 h | 500 h | 750 h | 1000 h |
|---|---|---|---|---|---|
| Äthylen | Gew.% | 11,0 | 8,8 | 7,3 | 6,5 |
| Propylen | " | 50,2 | 50,8 | 49,4 | 47,2 |
| Butylene | " | 20,6 | 21,0 | 20,1 | 19,6 |
| zusammen(Gew.%) | | 81,8 | 80,6 | 76,8 | 73,3 |
| $C_1$- bis $C_4$-Paraffine (Gew.%) | | 5,1 | 4,5 | 4,7 | 5,3 |
| $C_5^+$-Kohlenwasserstoffe (Gew.%) | | 13,1 | 14,9 | 18,5 | 21,4 |
| Methanol-Umsatz (%) | | 99,99 | 99,99 | 99,50 | 95,20 |

Beispiel 3

Ebenfalls in der Anordnung und der isothermen Verfahrensführung des Beispiels 1 wird der Einfluß der Reaktionstemperatur auf die Ausbeute an den olefinischen Kohlenwasserstoffen Äthylen, Propylen und Butylenen untersucht. Das Methanol-Wasser-Gemisch wurde im einmaligen Durchgang über den Katalysator geleitet; die Eintrittstemperatur stimmte mit der Reaktionstemperatur überein. Ergebnisse:

| Reaktionstemperatur: | | $420^{\circ}C$ | $480^{\circ}C$ |
|---|---|---|---|
| Äthylen | (Gew.%) | 11,5 | 11,0 |
| Propylen | " | 31,9 | 50,2 |
| Butylene | " | 19,8 | 20,6 |
| zusammen (Gew.%) | | 63,2 | 81,8 |
| $C_1$- bis $C_4$-Paraffine (Gew.%) | | 11,5 | 6,1 |
| $C_5^+$-Kohlenwasserstoffe " | | 26,8 | 13,1 |

Die besten Ergebnisse wurden bei der Reaktionstemperatur von $480^{\circ}C$ erreicht. Oberhalb von etwa $500^{\circ}C$ tritt thermische Crackung auf, welche die Ausbeute an Äthylen, Propylen und Butylenen verringert.

Patentansprüche

1) Verfahren zur Herstellung von Olefinen mit 2 bis 5 C-Atomen aus Methanol, das man bei Temperaturen von 200 bis 600°C und einem Druck von 1 bis 35 bar an einem Silicium enthaltenden Katalsator umsetzt und ein olefinhaltiges Produkt abzieht, dadurch gekennzeichnet, daß als Katalysator kristallines Siliciumdioxid ohne Ionenaustauschfähigkeit (Silicalite) verwendet wird.

2) Verfahren nach Anspruch 1, dadurch gekennzeichnet, daß Methanol als Einsatzstoff ganz oder teilweise durch Dimethylether oder niedere Alkohole ersetzt ist.

3) Verfahren nach Anspruch 1 oder 2, dadurch gekennzeichnet, daß das Produkt der Umsetzung zu mindestens 75 Vol.% aus $C_2$- bis $C_5$-Olefinen besteht.

4) Verfahren nach Anspruch 1 oder einem der folgenden, dadurch gekennzeichnet, daß das Produkt pro kg $C_2H_4$ mindestens 2,5 kg $C_3H_6$ enthält.

5) Verfahren nach Anspruch 1 oder einem der folgenden, dadurch gekennzeichnet, daß das Produkt pro kg $C_2H_4$ mindestens 1 kg $C_4H_8$ enthält.

6) Verfahren nach Anspruch 1 oder einem der folgenden, dadurch gekennzeichnet, daß der Einsatzstoff ohne Rückführung von Restgasen und/oder Einsatzstoff umgesetzt und das Methanol zu mindestens 80% zu $C_2$- bis $C_5$-Olefinen umgesetzt wird.

7) Verfahren nach Anspruch 1 oder einem der folgenden, dadurch gekennzeichnet, daß man die Umsetzung am Silicalite-Katalysator adiabatisch durchführt.

8) Verfahren nach Anspruch 1 oder einem der folgenden, dadurch gekennzeichnet, daß das olefinhaltige Produkt den Katalysator mit Temperaturen im Bereich von 350 bis 550°C verläßt.

**Europäisches Patentamt**

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. $^3$) |
|---|---|---|---|
| X,P | EP-A-0 100 604 (SUMMIT GAS SYSTEMS PTE) <br> * Anspruch 1; Beispiel 1 * <br> --- | 1,2 | C 07 C 1/24 <br> C 07 C 11/02 <br> C 10 G 3/00 |
| X | EP-A-0 035 807 (SHELL INTERNATIONALE RESEARCH MAATSCHAPPIJ) <br> * Ansprüche 1-5 * <br> --- | 1,2 | |
| A | GB-A-2 106 132 (CHEVRON RESEARCH) <br> * Anspruch 1; Figuren 1, 2 * <br> --- | 1 | |
| A | GB-A-2 106 131 (CHEVRON RESEARCH) <br> * Anspruch 1; Figuren 1, 2 * <br> --- | 1 | |
| A | US-A-4 320 241 (T.C. FRANKIEWICZ) <br> * Ansprüche 1, 3 * <br><br> ----- | 1 | |

**RECHERCHIERTE SACHGEBIETE (Int. Cl. $^3$)**

C 07 C 1/20
C 07 C 1/24
C 07 C 11/02
C 07 C 11/04
C 07 C 11/06
C 07 C 11/08
C 07 C 11/09
C 07 C 11/10
C 10 G 3/00

Der vorliegende Recherchenbericht wurde für alle Patentansprüche erstellt.

| Recherchenort <br> BERLIN | Abschlußdatum der Recherche <br> 16-08-1984 | Prüfer <br> KNAACK M |
|---|---|---|